# FASCICULE DE BREVET EUROPEEN

(11) **EP 0 981 301 B1**
(45) Date de publication et mention de la délivrance du brevet: **20.08.2003**
(21) Numéro de dépôt: 98914942.2
(22) Date de dépôt: 18.03.1998
(51) Int. Cl.: A61B 17/70

(54) **IMPLANT POUR DISPOSITIF D'OSTEOSYNTHESE ET OUTIL DE MONTAGE D'UN TEL IMPLANT**
IMPLANTAT FÜR OSTEOSYNTHESEVORRICHTUNG UND WERKZEUG ZUM EINSETZEN EINES SOLCHEN
IMPLANT FOR OSTEOSYNTHESIS DEVICE AND TOOL FOR SETTING SUCH IMPLANT

(30) Priorité: 18.03.1997 FR 9703277
(43) Date de publication de la demande: 01.03.2000
(73) Titulaire: SCIENT'X, 75007 Paris (FR)
(72) Inventeur: ALBY, Albert, F-75016 Paris (FR)
(74) Mandataire: Thibault, Jean-Marc
(86) Numéro de dépôt international: FR9800543
(87) Numéro de publication internationale: WO98041159

(56) Documents cités:
- EP-A- 0 528 706
- EP-A- 0 682 918
- WO-A-93/11715
- DE-A- 19 605 640

## Description

La présente invention concerne un implant pour dispositif d'ostéosynthèse, notamment du rachis, comprenant un dispositif d'ancrage osseux surmonté d'une tête de fixation constituée par deux branches latérales formant un U ouvert et destiné à recevoir une tige de liaison en vue de son immobilisation par serrage par l'intermédiaire d'un écrou fileté, apte à se visser sur des parties filetées correspondantes réalisées sur les parois extérieures des branches latérales de la tête de fixation.

Différents implants dont le corps de fixation comprend des branches latérales formant un canal pour recevoir une tige de liaison ont été décrits.

### TECHNIQUE ANTERIEURE

Ainsi, dans la demande de brevet français n° 2 711 909 les branches latérales du U formant la tête sont filetées, non seulement extérieurement, mais également intérieurement, afin de recevoir un bouchon conique destiné à agir en écartement sur les branches pour immobiliser l'écrou.

Dans ce dispositif, il est nécessaire d'engager à la main l'écrou sur le filetage extérieur de la tête, puis le bouchon dans le filetage interne du dispositif d'ancrage osseux en place sur le patient avant d'effectuer leur blocage définitif par l'intermédiaire de deux outils appropriés distincts.

Ce dispositif de conception relativement simple est facile à mettre en oeuvre, mais il est nécessaire d'utiliser deux outils et d'engager préalablement à la main l'écrou et le bouchon dans leur filetage correspondant, ce qui est réalisé avec un certain tâtonnement et dans des conditions défavorables en raison de l'exécution au cours d'une intervention chirurgicale.

On a proposé, dans la demande de brevet européen n° 0 528 706 ou encore dans le brevet français n° 2 658 414 d'effectuer le serrage de la tige par l'intermédiaire d'un élément d'appui annexe destiné à être préalablement inséré entre les branches de la tête de fixation pour assurer un meilleur contact avec la tige.

Dans ce cas, la difficulté supplémentaire consiste dans la manipulation de l'élément d'appui intermédiaire qu'il est nécessaire d'insérer entre les branches parallèles de la tête de fixation.

On comprend bien que cette difficulté de centrage de l'élément d'appui serait encore augmentée si l'élément d'appui était rendu solidaire de l'écrou puisque le praticien devrait à la fois rechercher à tâtons l'indexation de l'élément d'appui, caché par l'écrou, et visser en aveugle l'écrou sur la tête.

L'invention a pour but de remédier aux différents inconvénients précités en proposant un dispositif permettant une fixation solide et une mise en place simple et facile d'un élément d'appui pour une tige de liaison.

### EXPOSE DE L'INVENTION

L'invention concerne un implant pour dispositif d'ostéosynthèse du rachis comprenant un dispositif d'ancrage osseux surmonté d'une tête de fixation constituée par deux branches latérales formant un U ouvert et destiné à recevoir une tige de liaison en vue de son immobilisation par serrage, par l'intermédiaire d'un écrou fileté apte à se visser sur des parties filetées correspondantes réalisées sur les parois extérieures, partiellement cylindriques, des branches latérales de la tête de fixation, caractérisé en ce que l'écrou comporte, dans sa zone diamétrale, un patin monté libre en rotation.

La largeur du patin est adaptée pour permettre le coulissement dudit patin entre les branches de la tête de fixation en délimitant deux dégagements latéraux de part et d'autre dudit patin pour le passage des branches du U à l'intérieur de l'écrou et l'insertion des deux broches d'un outil auxiliaire de préhension de l'écrou pour faciliter son montage sur la tête de fixation.

La tête de fixation comporte deux rainures réalisées en vis-à-vis sur les parois internes de ces branches en U pour assurer, après montage de la tige, le guidage des broches de l'outil sur la tête et, conséquemment, une indexation en aveugle du patin entre les branches du U de ladite tête, avant et pendant le serrage de l'écrou qui le supporte, le serrage s'effectuant par l'intermédiaire du même outil.

La présente invention concerne également les caractéristiques qui ressortiront au cours de la description qui va suivre et qui devront être considérées isolément ou selon toutes leurs combinaisons techniques possibles.

### BREVE DESCRIPTION DES DESSINS

Cette description, donnée à titre d'exemple non limitatif, fera mieux comprendre comment l'invention peut être réalisée, en référence aux dessins annexés sur lesquels :
- la **fig. 1** est une vue éclatée à échelle double, d'un implant selon une forme de réalisation de l'invention et d'un outil associé destiné à son montage, les vues 1A, 1B, 1C représentant respectivement, un dispositif d'ancrage, un écrou, un outil dont seule l'extrémité a été représentée ;
- la **fig. 2** est une vue selon une coupe longitudinale de la **fig. 1,** les vues 2A, 2B, 2C représentant respectivement, le dispositif d'ancrage, l'écrou, l'outil ;
- la **fig. 3** est une vue de dessus du dispositif d'ancrage ;
- la **fig. 4** est une vue de dessus de l'écrou ;
- la **fig. 5** est une vue de côté de l'écrou dont la moitié est une coupe selon la ligne V-V de la **fig. 4 ;**
- la **fig. 6** est une vue à échelle 1, d'un outil de montage, représenté partiellement en coupe.

### MEILLEURE MANIERE DE REALISER L'INVENTION

L'implant représenté par les **fig. 1A** et **1B** est constitué d'un dispositif d'ancrage osseux **1** comprenant une vis pédiculaire autotaraudeuse connue en soi, se composant d'une partie filetée **2** à pas de vis large, destiné à se visser dans l'os, et une partie supérieure apparente constituant une tête de fixation **3** pour une tige **4** de liaison entre deux dispositifs d'ancrage osseux **1** successifs.

La tête de fixation **3** est constituée par deux branches latérales **5** formant un U ouvert destiné à recevoir la tige de liaison **4**.

Les faces externes **5a** des branches **5** s'inscrivent dans un cylindre et sont filetées pour recevoir un écrou fileté correspondant **6** apte à se visser sur la tête **3** en vue d'agir en serrage sur la tige **4**, une fois en place.

La tige **4** est cylindrique et elle est également moletée sur toute sa surface externe, de manière à éviter les glissements après serrage et pour améliorer l'immobilisation de ladite tige.

Le fond **7** du U formé par la tête de fixation **3** est de rayon sensiblement correspondant à celui de la tige **4** et se trouve également moleté.

L'écrou de serrage **6** est de manière connue en soi, de section externe hexagonale **6a** pour permettre sa préhension par un outil correspondant et comporte une partie interne cylindrique filetée ou taraudage **6b** destinée à être vissée sur la partie filetée de la tête **3** du dispositif d'ancrage osseux **1.**

L'écrou **6** comporte, dans sa zone diamétrale, un patin **8** monté libre en rotation, dont la largeur **L** est telle qu'elle permette son coulissement axial entre les branches **5** du U de la tête de fixation **3,** tout en délimitant par rapport audit écrou **6,** deux dégagements latéraux **9** destinés, d'une part, au passage des branches **5** de la tête **3,** lors du vissage de l'écrou **6** et, d'autre part, à l'insertion de deux broches **11** d'un outil auxiliaire de préhension **10** dudit écrou **6,** pour faciliter son montage sur la tête de fixation **3.**

Les parois internes **5b** des branches **5** en vis-à-vis de la tête de fixation **3,** comportent deux rainures **12** au centre desdites parois.

Ces rainures servent à assurer, après mise en place de la tige **4**, le guidage des broches **11** de l'outil **10** sur la tête **3** et, conséquemment, une indexation en aveugle du patin **8** entre les branches **5** du U de la tête **3,** avant et pendant le serrage de l'écrou **6** qui le supporte. Le serrage de l'écrou **6** s'effectue par l'intermédiaire du même outil **10.**

Selon une autre caractéristique de l'invention, le patin **8** comporte, sur ses bords latéraux **8a**, deux encoches **13** faisant partie chacune d'un dégagement latéral **9.** Les encoches **13** délimitent, en complément et en coopération avec les rainures de guidage **12** des branches **5** de la tête de fixation **3,** des logements **12/13** destinés à l'introduction et au positionnement des broches **11** de l'outil **10.** Comme illustré sur les dessins, la largeur **L** du patin **8** prise perpendiculairement à la direction diamétrale de l'écrou **6**, présente une valeur par exemple égale, en dehors des encoches **13,** sensiblement à l'écart entre les branches **5** de la tête de fixation **3.**

Selon une autre caractéristique de l'invention, la surface du patin **8b** est concave et congruente de la surface de la tige cylindrique **4** et moletée pour une meilleure adhérence sur ladite tige **4**.

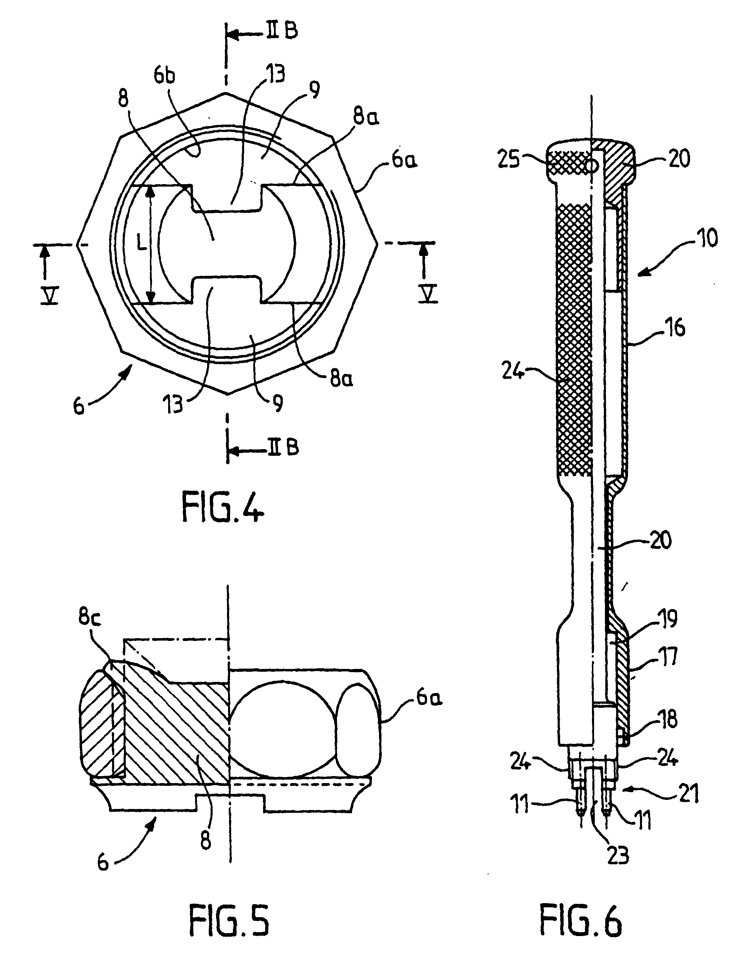

Le patin **8** est fixé de manière libre en rotation sur l'écrou **6** par un sertissage adéquat **8c (fig. 5)**.

Selon l'exemple de réalisation représenté, le dispositif d'ancrage osseux est une vis pédiculaire mais, bien entendu, il pourra s'agir de tout autre moyen comme par exemple un crochet.

Il est également à noter que tous les éléments qui viennent d'être décrits et constituant l'implant, sont à réaliser en alliage de titane ou matériau équivalent dont les propriétés amagnétiques n'engendrent pas d'artefacts lors d'examens à l'IRM ou au scanner.

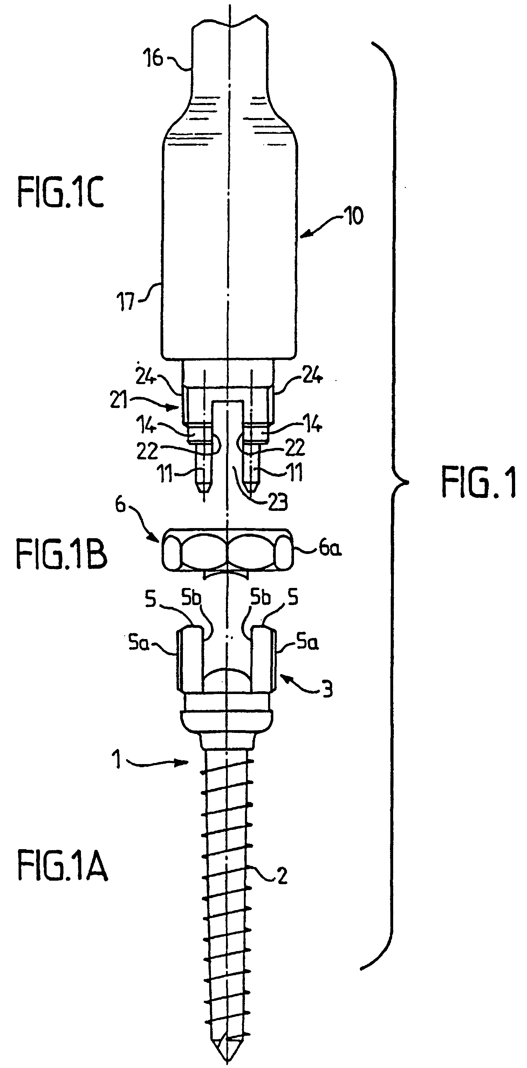

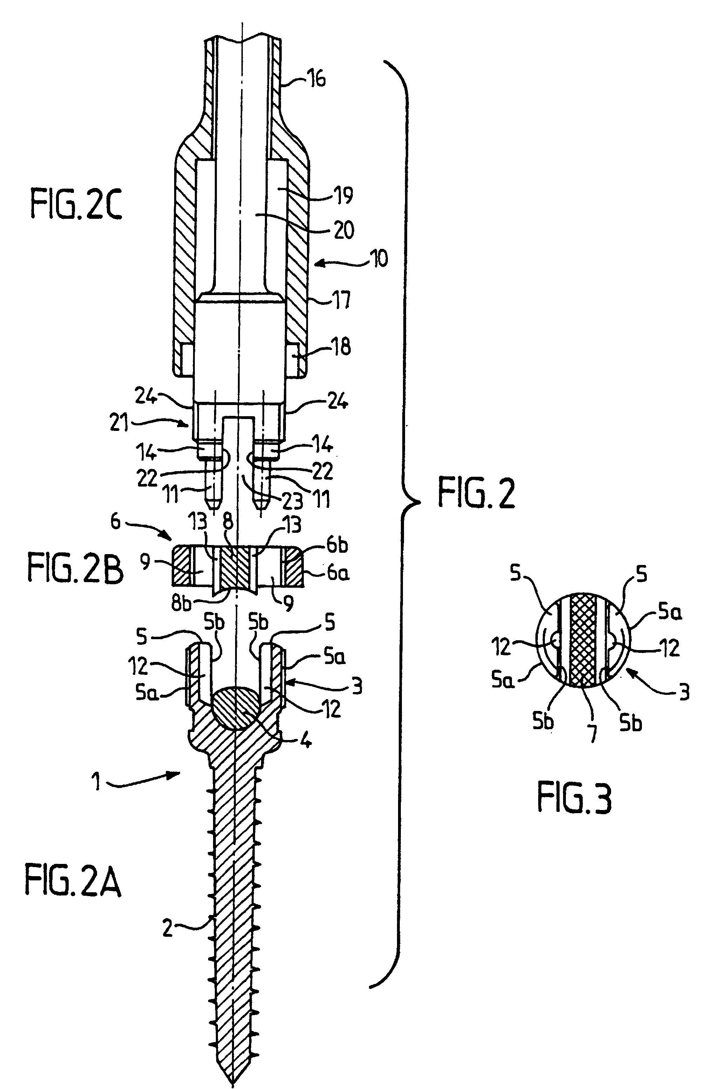

L'invention concerne également la structure particulière de l'outil auxiliaire **10.** Comme le montrent particulièrement bien les **fig. 1C, 2C** et **6,** ledit outil est constitué par un fourreau cylindrique **16** comportant une partie d'extrémité **17**, formant un embout hexagonal femelle **18**, apte à coopérer avec l'écrou **6** de l'implant et dans lequel débouche un alésage **19** destiné au coulissement axial d'un organe coulissant **20.**

L'organe coulissant **20** comporte une extrémité sortante **21** constituée par deux branches latérales **22**, partiellement cylindriques, formant un U ouvert et dont les parois extérieures **24** sont filetée de manière à permettre le vissage préalable de l'écrou **6** sur l'outil, tout en permettant le libre coulissement du patin **8** dudit écrou entre lesdites branches filetées **22** de ce même outil **10**.

De cette manière, l'écrou **6**, préalablement vissé sur les branches **22** de l'outil **10,** peut, après son positionnement sur la tête de fixation **3** par l'intermédiaire des broches **11** dans les rainures **12** des branches **5** de ladite tête, être transféré directement du filetage **24** de l'outil **10** au filetage **5a** de la tête **3.** Le transfert s'effectue par l'intermédiaire de l'embout hexagonal **18** actionné en rotation par l'opérateur, tout en immobilisant l'organe coulissant également par rotation.

L'actionnement en rotation de l'écrou **6** s'effectuera jusqu'à son blocage sur la tige **4** par l'intermédiaire du patin **8**.

Pour une meilleure préhension de l'outil **10**, la zone supérieure **26** du fourreau cylindrique **16** et l'extrémité supérieure **25** de l'organe coulissant **20** sont moletées.

## Revendications

1. Implant pour dispositif d'ostéosynthèse notamment du rachis comprenant un dispositif d'ancrage osseux **(1)** surmonté d'une tête de fixation **(3)** constituée par deux branches latérales **(5)** formant un U ouvert et destiné à recevoir une tige de liaison **(4)** en vue de son immobilisation par serrage, par l'intermédiaire d'un écrou fileté **(6)** apte à se visser sur des parties filetées correspondantes réalisées sur les parois extérieures **(5a),** partiellement cylindriques, des branches latérales de la tête de fixation **(3)**, **caractérisé en ce que** l'écrou **(6)** comporte, dans sa zone diamétrale, un patin **(8)** monté libre en rotation.

2. Implant pour dispositif d'ostéosynthèse selon la revendication 1, **caractérisé en ce que** la largeur **(L)** du patin **(8)** est adaptée pour permettre le coulissement dudit patin entre les branches **(5)** de la tête de fixation en délimitant deux dégagements latéraux **(9)** de part et d'autre dudit patin **(8)** pour permettre le passage des branches **(5)** du U à l'intérieur de récrou **(6)** et l'insertion de deux broches **(11)** d'un outil auxiliaire de préhension **(10)** de récrou **(6)** pour faciliter son montage sur la tête de fixation **(3).**

3. Implant pour dispositif d'ostéosynthèse selon l'une des revendications 1 ou 2, **caractérisé en ce que** la tête de fixation **(3)** comporte deux rainures **(12)** réalisées en vis-à-vis sur les parois internes **(5b)** des branches en U **(5)** pour assurer, après montage de la tige **(4),** le guidage de broches **(11)** d'un outil **(10)** sur la tête de fixation **(3)** et une indexation en aveugle du patin **(8)** entre les branches en U **(5)** de ladite tête **(3),** avant et pendant le serrage de l'écrou **(6)** qui le supporte, par l'intermédiaire du même outil **(10).**

4. Implant pour dispositif d'ostéosynthèse selon la revendication 3, **caractérisé en ce que** le patin **(8)** comporte, sur ses bords latéraux **(8a),** deux encoches **(13)** délimitant, en complément et en coopération avec les rainures de guidage **(12)** des branches en U **(5)** de la tête de fixation **(3),** des logements **(12/13)** destinés à l'introduction et au positionnement de broches **(11)** d'un outil **(10).**

5. Implant pour dispositif d'ostéosynthèse selon la revendication 4, **caractérisé en ce que** les encoches **(13)** forment, dans les bords **(8a)** du patin **(8),** des chicanes quadrangulaires destinées au logement des prolongements **(14)** de broches **(11)** d'un outil **(10).**

6. Implant pour dispositif d'ostéosynthèse selon l'une des revendications 4 ou 5, **caractérisé en ce que** la surface **(8b)** du patin **(8)** est concave et congruente de la surface de la tige cylindrique **(4)** et moletée pour une meilleure adhérence sur ladite tige **(4).**

7. Outil de montage de l'implant pour dispositif d'ostéosynthèse selon l'une des revendications précédentes, **caractérisé en ce qu'**il est constitué par un fourreau cylindrique **(16)** comportant une partie d'extrémité **(17)** formant un embout hexagonal femelle **(18),** d'une part, apte à coopérer avec l'écrou **(6)** de l'implant et, d'autre part, dans lequel débouche un alésage **(19)** destiné au coulissement axial d'un organe coulissant **(20)** et **en ce que** ledit organe coulissant **(20)** comporte une extrémité sortante **(21)** constituée par deux branches latérales **(22),** partiellement cylindriques, formant un U ouvert et dont les parois extérieures **(24)** sont filetée de manière à permettre le vissage de l'écrou **(6)** sur l'outil, tout en permettant le libre coulissement du patin **(8)** dudit écrou **(6)** entre les branches filetées **(22)** de l'outil **(10),** de manière à ce que l'écrou **(6),** préalablement vissé sur les branches **(22)** de l'outil **(10),** puisse, après son positionnement sur la tête de fixation **(13)** par l'intermédiaire de ses broches **(11)** dans les rainures **(12)** des branches en U **(5)** de ladite tête **(3),** être transférés directement du filetage **(24)** de l'outil **(10)** au filetage **(5a)** de la tête **(3)** par l'intermédiaire de l'embout hexagonal **(18)** actionné en rotation par l'opérateur, tout en immobilisant l'organe coulissant **(20)** également par rotation.

## Patentansprüche

1. Implantat für eine Vorrichtung zur Osteosynthese insbesondere der Wirbelsäule, mit einer Vorrichtung zur Knochenverankerung (1), über der ein Befestigungskopf (3) angeordnet ist, der aus zwei seitlichen Armen (5) gebildet ist, die ein offenes U bilden und dazu bestimmt sind, eine Verbindungsstange (4) aufzunehmen, um diese durch Festklemmen mittels einer Gewindemutter (6) zu fixieren, die auf die entsprechenden Gewindeteile geschraubt zu werden vermag, die an den teilweise zylindrischen Außenwänden (5a) der Seitenarme des Befestigungskopfes (3) ausgeführt sind,
**dadurch gekennzeichnet, dass** die Mutter (6) in ihrem diametralen Bereich ein Gleitstück (8) umfasst, das drehbeweglich montiert ist.

2. Implantat für eine Vorrichtung zur Osteosynthese nach Anspruch 1,
**dadurch gekennzeichnet, dass** die Breite (L) des Gleitstücks (8) so angepasst ist, dass das Gleiten des Gleitstücks zwischen den Armen (5) des Befestigungskopfs möglich ist, um das Einführen der U-förmigen Arme in die Mutter (6) und das Einfügen von zwei Stiften (11) eines Hilfswerkzeugs (10) zum Ergreifen der Mutter (6) zu ermöglichen, um deren Befestigung auf dem Befestigungskopf (3) zu erleichtern.

3. Implantat für eine Vorrichtung zur Osteosynthese nach einem der Ansprüche 1 oder 2, **dadurch gekennzeichnet, dass** der Befestigungskopf (3) zwei Nuten (12) umfasst, die einander gegenüberliegend an den Innenwänden (5b) der U-förmigen Arme (5) ausgeführt sind, um nach der Montage der Stange (4) die Führung der Stifte (11) eines Werkzeugs (10) auf dem Befestigungskopf (3) und ein blindes Einführen des Gleitstücks (8) zwischen den U-förmigen Armen (5) des Kopfs (3) vor und während des Festklemmens der diesen tragenden Mutter (6) mittels desselben Werkzeugs (10) zu gewährleisten.

4. Implantat für eine Vorrichtung zur Osteosynthese nach Anspruch 3,
**dadurch gekennzeichnet, dass** das Gleitstück (8) an seinen Seitenrändern (8a) zwei Aussparungen (13) aufweist, die komplementär zu und in Zusammenwirkung mit den Führungsnuten (12) der U-förmigen Arme (5) des Befestigungskopfs (3) Aufnahmen (12/13) begrenzen, die zum Einführen und Positionieren der Stifte (11) eines Werkzeugs bestimmt sind.

5. Implantat für eine Vorrichtung zur Osteosynthese nach Anspruch 4,
**dadurch gekennzeichnet, dass** die Aussparungen (13) in den Rändern (8a) des Gleitstücks (8) viereckige Schikanen bilden, die zum Lagern von Verlängerungen (14) der Stifte (11) eines Werkzeugs (10) bestimmt sind.

6. Implantat für eine Vorrichtung zur Osteosynthese nach einem der Ansprüche 4 oder 5,
**dadurch gekennzeichnet, dass** die Oberfläche (8b) des Gleitstücks (8) konkav und zu der Oberfläche des zylindrischen Stange (4) kongruent ist und zwecks einer besseren Anhaftung an der Stange (4) geriffelt ist.

7. Werkzeug zur Montage des Implantats für Vorrichtung zur Osteosynthese nach einem der vorhergehenden Ansprüche,
**dadurch gekennzeichnet, dass** es von einer zylindrischen Hülse (16) gebildet ist, mit einem Endteil (17), das eine sechseckige Steckbuchse (18) bildet, die zum einen mit der Mutter (6) des Implantats zusammenzuwirken vermag und in die zum anderen eine Bohrung (19) mündet, die zum axialen Gleiten eines Gleitorgans (20) bestimmt ist, und dass das Gleitorgan (20) ein vorstehendes Endstück (21) umfasst, das von zwei teilweise zylindrischen, ein offenes U bildenden Seitenarmen (22) gebildet ist, deren Außenwände (24) mit Gewinde in der Art versehen sind, dass das Festschrauben der Mutter (6) zwischen den Gewindearmen (22) des Werkzeugs (10) möglich ist, so dass die vorher auf die Arme (22) des Werkzeugs (10) geschraubte Mutter (6), nachdem diese mittels ihrer Stifte (11) auf dem Befestigungskopf (13) in den Nuten (12) der U-förmigen Arme (5) des Kopfes (3) positioniert ist, direkt von dem Gewinde (24) des Werkzeugs (10) auf das Gewinde (5a) des Kopfs (3) durch die sechseckige Steckbuchse (18) übertragen werden kann, die durch den Operateur in Drehung versetzt wird, wobei jedoch das Gleitorgan (20) durch Drehung fixiert wird.

## Claims

1. An implant for an osteosynthesis device, in particular for the spine, the implant comprising a bone anchor device (1) surmounted by a fixing head (3) constituted by two side branches (5) forming an open U-shape for receiving a link rod (4) that is to be held by being clamped by means of a threaded nut (6) suitable for screwing onto corresponding threaded portions formed on the partially-cylindrical outside walls (5a) of the side branches of the fixing head (3), the implant being **characterized in that** the diametral zone of the nut (6) includes a pad (8) that is mounted free to rotate.

2. An osteosynthesis device implant according to claim 1, **characterized in that** the width (L) of the pad (8) is adapted to enable said pad to slide between the branches (5) of the fixing head, defining two side gaps (9) on either side of said pad (8) to allow the branches (5) of the U-shape to pass inside the nut (6) and to receive two pins (11) of an auxiliary tool (10) for gripping the nut (6) in order to facilitate mounting thereof on the fixing head (3).

3. An osteosynthesis device implant according to claim 1 or claim 2, **characterized in that** the fixing head (3) includes two grooves (12) facing each other in the inside walls (5b) of the branches (5) of the U-shape to act, after the rod (4) has been mounted, to guide the pins (11) of a tool (10) over the fixing head (3) and to enable the position of the pad (8) between the branches (5) of the U-shape of said head (3) to be indexed blind before and during tightening of the nut (6) supporting the pad, tightening being performed by means of the same tool (10).

4. An osteosynthesis device implant according to claim 3, **characterized in that** the sides (8a) of the pad (8) include two notches (13) in addition to and for co-operating with the guide grooves (12) in the branches (5) of the U-shape of the fixing head (3) to provide housings (12/13) for receiving and positioning pins (11) of a tool (10).

5. An osteosynthesis device implant according to claim 4, **characterized in that** the notches (13) in the sides (8a) of the pad (8) form quadrangular baffles for receiving extensions (14) of pins (11) of a tool (10).

6. An osteosynthesis device implant according to claim 4 or claim 5, **characterized in that** the surface (8b) of the pad (8) is concave and complementary to the surface of the cylindrical rod (4), and is knurled to adhere better on said rod (4).

7. A tool for mounting an osteosynthesis device implant according to any preceding claim, the tool being **characterized in that** it is constituted by a cylindrical sheath (16) having an end portion (17) forming a female hexagonal socket (18) firstly suitable for co-operating with the nut (6) of the implant, and secondly having a bore (19) opening out therein for receiving a sliding member (20) to slide axially, and **in that** said sliding member (20) has a projecting end (21) constituted by two partially-cylindrical side branches (22) forming an open U-shape with outside walls (24) that are threaded so as to enable the nut (6) to be screwed onto the tool while allowing the pad (8) of said nut (6) to slide freely between the threaded branches (22) of the tool (10) in such a manner that the nut (6), initially screwed onto the branches (22) of the tool (10), is capable, once positioned on the fixing head (13) by means of its pins (11) penetrating in the grooves (12) of the branches (5) of the U-shape of said head (3), of being transferred directly from the thread (24) on the tool (10) to the thread (5a) on the head (3) by the operator turning the hexagonal socket (18) while also preventing the sliding member (20) from turning.
